# EUROPÄISCHE PATENTSCHRIFT

(11) **EP 4 166 175 B1**
(45) Veröffentlichungstag und Bekanntmachung des Hinweises auf die Patenterteilung: **04.12.2024**
(21) Anmeldenummer: 22196314.3
(22) Anmeldetag: 19.09.2022
(51) Int. Cl.: A61M 16/00

(54) **KOPPLUNG ZWISCHEN ANTRIEB UND GEBLÄSEKOPF VON EINEM BEATMUNGSGERÄT**
COUPLING BETWEEN DRIVE AND FAN HEAD OF A BREATHING DEVICE
ACCOUPLEMENT ENTRE UN ENTRAÎNEMENT ET UNE TÊTE DE VENTILATEUR D'UN DISPOSITIF RESPIRATOIRE

(30) Priorität: 16.10.2021 DE 102021005180
(43) Veröffentlichungstag der Anmeldung: 19.04.2023
(73) Patentinhaber: Löwenstein Medical Technology S.A., 2557 Luxembourg (LU)
(72) Erfinder: SCHREGEL, Christian-Georg, 61169 Friedberg (DE)
(74) Vertreter: Marx, Thomas

(56) Entgegenhaltungen:
- EP-B1- 1 170 025
- WO-A1-2013/096495
- DE-U1- 20 022 295
- US-A1- 2010 000 535
- US-A1- 2014 020 684
- US-A1- 2018 104 436
- US-B1- 6 672 300
- US-B2- 6 526 970

## Beschreibung

Die Erfindung betrifft ein Beatmungsgerät mit einer Kopplung zwischen einem Motor und einem Gebläsekopf. Insbesondere im klinischen Bereich eingesetzte Beatmungsgeräte bedürfen einer regelmäßigen hygienischen Aufbereitung. Diese hygienische Aufbereitung betrifft insbesondere die Teile des Beatmungsgerätes, welche das Patientengas fördern und leiten. In manchen Beatmungsgeräten kommt dabei unter anderem das Gebläse mit dem Patientengas in Berührung. In der Regel ist das Gebläse unmittelbar mit dem Motor verbunden, wodurch auch der Motor als mit Patientengas kontaminiert gilt. Im Stand der Technik ist bekannt, dass das gesamte Element mit Motor und Gebläse aus dem Beatmungsgerät zur Aufbereitung entfernt werden kann. Dadurch muss der Motor zu einer hygienischen Aufbereitung ausgelegt sein, beispielsweise durch widerstandsfähige Lager und entsprechenden elektronischen Komponenten. Trotz der speziell ausgewählten Materialien und Komponenten ist dennoch von einer stark reduzierten Lebenszeit des Motors auszugehen.

US 6672300 B1 zeigt ein Beatmungsgerät mit einem Gebläsekopf und einer Antriebsvorrichtung, wobei der Gebläsekopf und die Antriebsvorrichtung über eine kraftschlüssige Kopplung miteinander lösbar gekoppelt sind.

Die Erfindung wird durch die beigefügten Ansprüche definiert.

In manchen nicht beanspruchten Beispielen kennzeichnet das Beatmungsgerät, dass die Kopplung derart eingerichtet und ausgebildet ist, dass im Bereich der Kopplung kein Patientengas vom Gebläsekopf zur Antriebsvorrichtung gelangt.

In manchen nicht beanspruchten Beispielen kennzeichnet das Beatmungsgerät, dass die Kopplung zwischen der Antriebsvorrichtung und dem Gebläsekopf über mindestens zwei Koppelelemente erfolgt.

In manchen nicht beanspruchten Beispielen kennzeichnet das Beatmungsgerät, dass die Kopplung eine magnetische Kopplung ist. In manchen nicht beanspruchten Beispielen kennzeichnet das Beatmungsgerät, dass die Kopplung eine magnetische Verbindung ist.

In manchen nicht beanspruchten Beispielen kennzeichnet das Beatmungsgerät, dass die mindestens zwei Koppelelemente als mindestens zwei Koppelmagnete ausgebildet sind.

In manchen nicht beanspruchten Beispielen kennzeichnet das Beatmungsgerät, dass am Lüfterrad mindestens ein erster Koppelmagnet angeordnet ist und dass an der Motorachse mindestens ein zweiter Koppelmagnet angeordnet ist. In manchen nicht beanspruchten Beispielen kennzeichnet das Beatmungsgerät, dass die Koppelelemente eingerichtet und ausgebildet sind, eine magnetische Verbindung miteinander einzugehen.

In manchen nicht beanspruchten Beispielen kennzeichnet das Beatmungsgerät, dass das Gehäuse des Gebläsekopfes zumindest teilweise zwischen dem Koppelmagneten des Lüfterrades und dem Koppelmagneten der Motorachse angeordnet ist.

In manchen Ausführungsformen kennzeichnet das Beatmungsgerät, dass der Gebläsekopf als Verschleißteil ausgebildet ist

In manchen Ausführungsformen kennzeichnet das Beatmungsgerät, dass der Gebläsekopf als Aufbereitungsteil ausgebildet ist und für eine Aufbereitung in einem Thermodesinfektor und/oder einem Autoklav ausgelegt ist.

In manchen nicht beanspruchten Beispielen kennzeichnet das Beatmungsgerät, dass die Kopplung zwischen Antriebsvorrichtung und Gebläsekopf über einen Reibschluss erfolgt.

In manchen Ausführungsformen kennzeichnet das Beatmungsgerät, dass die Kopplung eine formschlüssige Verbindung ist, wobei die Koppelelemente, ein symmetrisches Kopplungsprofil aufweisen.

In manchen Ausführungsformen kennzeichnet das Beatmungsgerät, dass die Kopplung eine formschlüssige Verbindung ist. In manchen Ausführungsformen kennzeichnet das Beatmungsgerät, dass die Koppelelemente, ein asymmetrisches Kopplungsprofil aufweisen.

In manchen nicht beanspruchten Beispielen kennzeichnet das Beatmungsgerät, dass die Antriebsvorrichtung fest in dem Beatmungsgerät verbaut ist.

In manchen Ausführungsformen kennzeichnet das Beatmungsgerät, dass das Gebläseteil zur Aufbereitung in einem Thermodesinfektor und/oder Autoklaven ausgelegt ist.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen zumindest ausatmet und/oder einatmet.

Gemäß der Offenbarung ist der Gebläsekopf im Bereich der Kopplung hermetisch dicht von der Antriebsvorrichtung getrennt. Je nach Kopplungsart ist keine Durchführung für eine Lagerung und/oder eine Achse, welche mit dem Lüfterrad (auch als Impeller oder Laufrad bezeichnet) verbunden ist, durch das Gehäuse des Gebläsekopfes bzw. des entsprechenden Gebläseteils vorgesehen. Der Gebläsekopf ist beispielsweise unabhängig von der Antriebsvorrichtung aus dem Beatmungsgerät entfernbar und kann separat aufbereitet werden und/oder getauscht werden. Patientengas kann, zumindest im Bereich der Kopplung zwischen Antriebsvorrichtung und Lüfterrad, nicht vom Gebläseteil zur Antriebsvorrichtung gelangen.

Durch die Atemgasführung wird zumindest innerhalb des Beatmungsgerätes das Atemgas bzw. das Patientengas geleitet. Die gesamte Atemgasführung kann dabei in mehrere Teile unterteilt werden, welche, optional unabhängig voneinander, aus dem Beatmungsgerät entnommen werden können. Die Antriebsvorrichtung hingegen kann beispielsweise fest montiert im Beatmungsgerät verbleiben und muss nicht hygienisch aufbereitet werden. Die Auswahl an verfügbaren Komponenten und Materialien für die Antriebsvorrichtung kann so verbreitert werden. Es kann in manchen nicht beanspruchten Beispielen auch vorgesehen sein, dass die Antriebsvorrichtung ebenfalls separat (unabhängig von Gebläsekopf und/oder anderen Komponenten) entnommen werden kann.

Die Kopplung zwischen Antriebsvorrichtung und Gebläsekopf erfolgt beispielsweise magnetisch. Dazu ist auf der von der Antriebsvorrichtung angetriebenen Motorachse ein Koppelmagnet aufgebracht sowie an dem Lüfterrad ebenfalls ein korrespondierender Koppelmagnet. Das Lüfterrad ist im Gebläsekopf drehbar gelagert. Zwischen dem Koppelmagneten des Lüfterrades und dem Koppelmagneten der Motorachse verläuft, ohne Durchbruch/Durchführung, das Gehäuse des Gebläsekopfes bzw. des Gebläseteils. Die Kraftübertragung von der Antriebsvorrichtung auf das Lüfterrad erfolgt durch das Magnetfeld der Koppelmagneten.

Diese so mögliche, vollständige Trennung von Antriebsvorrichtung und Gebläsekopf ermöglicht eine Konstruktion des Gebläsekopfes und Komponenten des Gebläsekopfes rein aus aufbereitungsgeeigneten Materialien wie PA (Polyamid), PEEK (Polyetheretherketon), Keramik und Edelstahl.

Durch den Wegfall der Antriebsvorrichtung als aufzubereitende bzw. autoklavierbare Komponente kann die Konstruktion des Gebläseteils besser auf die Nutzung des zu Verfügung stehenden Raums in Thermodesinfektoren und Autoklaven angepasst werden. Da kein Zylinder für die Antriebsvorrichtung berücksichtigt werden muss kann die Formgebung des Kreisteils z.B. rein als flache, rechteckige Elemente stattfinden, die mit keinem ihrer Kanten die Dimensionen von einer Sterilisationseinheit (1 STE) überschreiten und auf eine kompakte Anordnung in der Aufbereitung ausgelegt sind. Im Gebläsekopf kann eine Verschleißauslegung auf kurzzeitige Nutzung stattfinden, um dem Kunden kostengünstige Einwegartikel zu Verfügung zu stellen falls dieser keine Aufbereitung vornehmen will oder der Gebläsekopf kann für lange Lebensdauer mit multiplen Aufbereitungen ausgelegt werden. Beide Varianten können dabei mit der gleichen Antriebsvorrichtung genutzt werden, so dass dem Kunden zu jedem Zeitpunkt die Wahl gegeben ist.

Auch können durch Veränderung der Form des Gehäuses vom Gebläsekopf und des Lüfterrads die Leistungscharakteristiken des Gebläses beeinflusst werden. Dadurch wird dem Anwender die Möglichkeit gegeben, durch einen simplen Austausch des Kreisteils die Leistungsdaten zu verändern. Zusätzlich kann auch vorgesehen sein, dass das Beatmungsgerät die verwendete Variante des Gebläsekopfes automatisch, beispielsweise elektronisch, erkennt.

Die Kopplung ist Teil eines Beatmungsgerätes Insbesondere kann das Beatmungsgerät ein Intensivbeatmungsgerät, ein klinisches Beatmungsgerät und/oder ein Anästhesiegerät sein. In manchen nicht beanspruchten Beispielen ist vorgesehen, dass das Patientengas zumindest teilweise in einem Kreislauf geführt wird. Der Patient atmet dabei über einen Inspirationszweig ein und in einen Exspirationszweig aus. Das ausgeatmete Patientengas wird beispielsweise so durch das Beatmungsgerät geführt, dass es - gegebenenfalls aufbereitet (mit frischem Sauerstoff versetzt, CO2-gefiltert, etc.) - wieder vom Patienten eingeatmet werden kann. Beispielsweise sorgt das Gebläse, zumindest umfassend einen Gebläsekopf mit Lüfterrad sowie eine Antriebsvorrichtung für das Lüfterrad, für eine Umwälzung bzw. Förderung des Patientengases im Kreislauf.

Das Beatmungsgerät ist dazu eingerichtet und ausgebildet ein Atemgas, auch Patientengas, von und/oder zu einem Patienten zu fördern. Dazu umfasst das Beatmungsgerät entsprechende Gasführungen, beispielweise in einem oder mehreren Gasführungsteilen angeordnet, sowie einen Inspirationsanschluss, einen Exspirationsanschluss. Zudem umfasst das Beatmungsgerät zumindest eine steuerbare Atemgasquelle, beispielsweise ein Gebläse. Das Gebläse umfasst zumindest einen Gebläsekopf mit Gehäuse und einem in dem Gehäuse gelagerten Lüfterrad. Alternativ oder ergänzend kann das Lüfterrad auch über ein Lüfterachse in der Gehäusewand des Gehäuses gelagert sein. Das Gebläse umfasst ferner zumindest eine Antriebsvorrichtung mit einer Motorachse. Die Antriebsvorrichtung ist beispielsweise ein Motor, etwa ein Elektromotor. Der Gebläsekopf ist beispielsweise separat von der Antriebsvorrichtung in einem Gebläseteil angeordnet. Das Beatmungsgerät wird durch eine Steuereinheit gesteuert. Die Steuereinheit ist unter anderem dazu eingerichtet, das Gebläse zu steuern. In manchen Ausfürhungsformen ist das Beatmungsgerät dazu eingerichtet, das Patientengas in einem Kreislauf zu führen, wobei der angeschlossene Patient Teil des Kreislaufs ist. Im Gasführungsteil angeordnet oder mit dem Gasführungsteil verbunden sind Ventile, Gasmischer, CO2-Absorber, etc. In manchen nicht beanspruchten Beispielen ist das Beatmungsgerät ein Anästhesiegerät. Ist das Beatmungsgerät ein Anästhesiegerät, so ist vorgesehen, dass auch Vorrichtungen zum Beimischen von Narkosemitteln zum Atemgas bzw. Patientengas mit dem Beatmungsgerät verbunden sind und/oder im Beatmungsgerät angeordnet sind.

Das Beatmungsgerät verfügt dabei über mindestens einen Teil, welcher entnehmbar ist. In manchen nicht beanspruchten Beispielen sind mindestens zwei Teile, ein Gebläseteil und ein Gasführungsteil, entnehmbar. Das Gebläseteil umfasst zumindest einen Gebläsekopf, welcher wiederum zumindest ein Lüfterrad umfasst, welcher innerhalb eines Gehäuses angeordnet ist. Das Gehäuse verfügt über zumindest zwei Gebläsestutzen, über welche das Gebläseteil bzw. der Gebläsekopf gasleitend mit dem Gasführungsteil verbunden werden kann. Das Gasführungsteil bildet beispielsweise die Verbindung, beispielsweise über interne Gasführungen, zwischen Gebläsekopf und dem Inspirationszweig und dem Exspirationszweig. Die Gasführungungen können dabei weitere Elemente wie Ventile und Anschlüsse, etwa zu einem CO2-Absorber, umfassen. Das Gebläseteil und das Gasführungsteil sind so ausgelegt, dass sie separat entnommen werden können. So ist beispielsweise eine separate hygienische Aufbereitung möglich.

Es ist beispielsweise vorgesehen, dass die Antriebsvorrichtung, beispielsweise ein Motor, im Beatmungsgerät fest verbaut ist. Die Kraftübertragung zwischen der Antriebsvorrichtung und dem Lüfterrad erfolgt beispielsweise über eine kraftschlüssige und/oder formschlüssige Verbindung. Beispielsweise ist vorgesehen, dass die Kraftübertragung über eine magnetische Kopplung erfolgt, wobei sowohl am Lüfterrad als auch an einer Motorachse der Antriebsvorrichtung jeweils mindestens ein Koppelmagnet angeordnet ist. Durch die magnetische Kopplung ist es möglich, dass die Gehäusewand des Gebläsekopfes bzw. des Gebläseteils zwischen dem Lüfterrad und dem Motor bzw. der Antriebsvorrichtung verlaufen kann, ohne dass ein Durchbruch und/oder eine Durchführung nötig ist. So kann beispielsweise erreicht werden, dass im Bereich der Kopplung kein Patientengas zum Motor bzw. der Antriebsvorrichtung gelangt. In manchen nicht beanspruchten Beispielen wird hier eine hermetisch geschlossene Dichtung erreicht.

Alternativ oder ergänzend kann die Kopplung von Antriebsvorrichtung und Lüfterrad über eine lösbare formschlüssige und/oder kraftschlüssige Verbindung erreicht werden. Beispielsweise weist dazu auch das Lüfterrad eine Achse (Lüfterachse) auf und sowohl an der Motorachse als auch an der Lüfterachse sind Koppelelemente angeordnet, über welche die beiden Achsen miteinander gekoppelt werden. Beispielsweise ist dabei an eine kraftschlüssige Verbindung gedacht, etwa über einen Reibschluss. Um eine solche kraftschlüssige Verbindung zu erreichen, wird das Gebläseteil beispielsweise im Beatmungsgerät lösbar fixiert, sodass über die Koppelelemente eine Kraftübertragung von Motorachse auf Lüfterachse stattfinden kann. Alternativ oder ergänzend kann auch eine lösbare formschlüssige Verbindung zwischen Motor und Lüfterrad vorgesehen sein. Dabei kann vorgesehen sein, dass die Kopplung über symmetrische oder asymmetrische Koppelelemente erfolgt.

Bei der Kopplung zwischen Antriebsvorrichtung und Lüfterrad über eine reibschlüssige Verbindung (Reibschluss) und/oder formschlüssige Verbindung muss die Lüfterachse durch das Gehäuse des Gebläseteils hindurchragen. Um eine Kontaminierung der Antriebsvorrichtung bzw. des Motors mit Patientengas zu verhindern ist vorgesehen, dass die Lüfterachse in der Gehäusewand des Gebläseteils drehbar und hermetisch dicht gelagert ist.

Es kann vorgesehen sein, dass eine Mehrzahl an verschiedenen Gebläseteilen, welche sich beispielsweise in der Art des Gebläsekopfes unterscheiden, zur Verfügung gestellt werden und in das Beatmungsgerät verbaut werden können. Es ist beispielsweise vorgesehen, dass die Kopplung so ausgelegt ist, dass jedes der Mehrzahl an Gebläseteilen mit der Antriebsvorrichtung verbindbar ist, ohne dass die Antriebsvorrichtung bzw. der Motor getauscht werden muss. In manchen nicht beanspruchten Beispielen ist das Beatmungsgerät dazu eingerichtet und ausgebildet, zu erkennen, welches Gebläseteil und/oder welcher Gebläsekopf verbaut ist. Beispielsweise kann dazu vorgesehen sein, dass im oder am Gebläseteil ein RFID-Chip oder ähnliches angeordnet ist, über welchen das Beatmungsgerät eine automatische Erkennung des Gebläseteils vornehmen kann. In manchen nicht beanspruchten Beispielen kann vorgesehen sein, dass über eine elektrische Anschlussleitung eine Erkennung des Gebläseteils bzw. des Gebläsekopfes erfolgen kann. Alternativ oder ergänzend kann vorgesehen sein, dass das Gebläseteil mit einem Barcode und/oder QR-Code ausgestattet ist, welcher über das Beatmungsgerät, optional automatisch, gescannt werden kann.

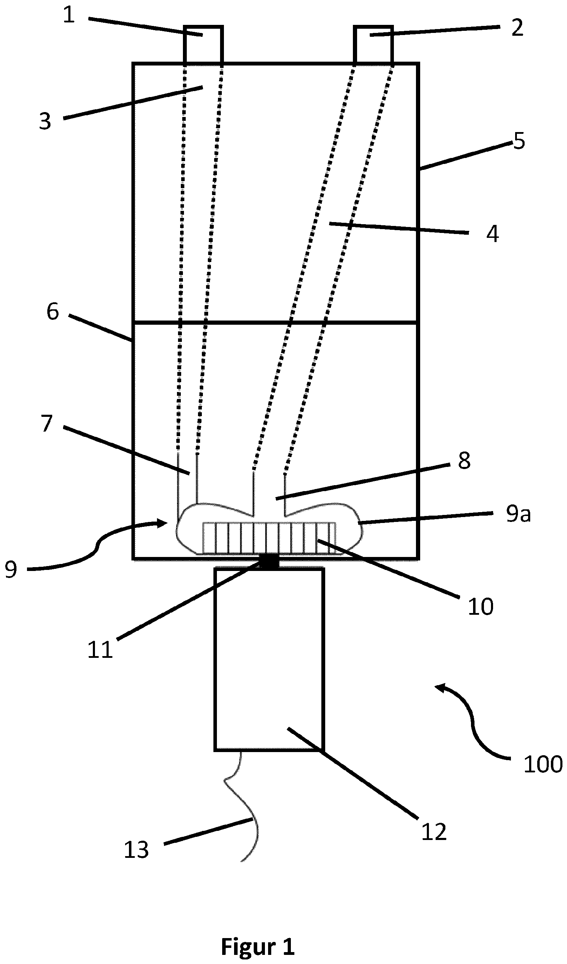

Das offenbarte System wird anhand der Figuren 1 bis 5 beispielhaft näher beschrieben.

Ein beispielhafter Ausschnitt aus einem Beatmungsgerät 100 mit einer Kopplung 11 und entnehmbarem Gasführungsteil 5 sowie entnehmbarem Gebläseteil 6 ist schematisch in **Figur 1** gezeigt.

Der Gasweg durch das Beatmungsgerät 100 ist durch den Gasführungsteil 5 und das Gebläseteil 6 dargestellt. Durch den Exspirationszweig 1 gelangt das vom Patienten ausgeatmete Patientengas in die Gasführung 3 im Gasführungsteil 5. Die Gasführung 3 umfasst beispielhaft Ventile sowie zumindest einen Anschluss und/oder Führung durch einen CO2-Absorber zur Entfernung von CO2 aus dem ausgeatmeten Patientengas. Durch die Gasführung 3 gelangt das Patientengas zum Gebläseteil 6, welcher zumindest den Gebläsekopf 9 umfasst. Die Gasführung 3 wird über den Gebläsestutzen 7 mit dem Gebläsekopf 9 verbunden. Der Gebläsekopf 9 umfasst zumindest ein Lüfterrad 10, welches in einem Lager im Gehäuse 9a des Gebläsekopfes 9 gelagert ist.

Das Lüfterrad 10 wird über eine Antriebsvorrichtung, beispielsweise mit einem Motor, angetrieben. Die Kraftübertragung von der Antriebsvorrichtung 12 zum Lüfterrad 10 erfolgt dabei über die Kopplung 11. Durch das Gebläse, bestehend aus Antriebsvorrichtung und Gebläsekopf 9, wird das Patientengas durch den Gebläsestutzen 8 in die Gasführung 4 und zum Inspirationszweig 2 gefördert. Durch den Inspirationszweig 2 gelangt das Patientengas zum Patienten. Die Gasführung 4 umfasst beispielsweise weitere Ventile sowie zumindest ein APL-Ventil. Auch kann vorgesehen sein, dass an oder in der Gasführung 4 Anschlüsse und Vorrichtungen angeordnet sind, über welche Gase, beispielsweise Sauerstoff, zum Patientengas zugemischt werden können. Ist das Beatmungsgerät 100 als Anästhesiegerät ausgebildet, kann auch eine Beimischung von Narkosemitteln zum Patientengas vorgesehen sein.

Es ist beispielhaft vorgesehen, dass sowohl das Gasführungsteil 5 als auch das Gebläseteil 6 separat aus dem Beatmungsgerät 100 entnommen werden können. In manchen nicht beanspruchten Beispielen kann auch vorgesehen sein, dass das Gasführungsteil 5 in mehrere Einzelteile unterteilt ist, welche unabhängig voneinander aus dem Beatmungsgerät 100 entnommen werden können. Beispielsweise ist so ein modularer Aufbau der Gasführung in dem Beatmungsgerät 100 möglich.

Auch ist vorgesehen, dass das Gebläseteil 6, unabhängig vom Gasführungsteil 5 und/oder dem Motor bzw. der Antriebsvorrichtung 12, entnehmen lässt. Das Gebläseteil 6 umfasst zumindest den Gebläsekopf 9 mit dem Lüfterrad 10. Damit sich der Gebläsekopf 9 von der Antriebsvorrichtung 12 bzw. dem Motor, trennen lässt, ist eine lösbare Kopplung 11 zwischen Antriebsvorrichtung 12 und Gebläsekopf 9 vorgesehen. Über die lösbare Kopplung 11 wird die Kraft der Antriebsvorrichtung 12 auf das Lüfterrad 10 übertragen. Für die Kopplung 11 kann eine magnetische Kopplung, eine kraftschlüssige Verbindung und/oder eine formschlüssige Verbindung zwischen Antriebsvorrichtung 12 und Lüfterrad 10 vorgesehen sein. Insbesondere ist vorgesehen, dass die Kopplung 11 so eingerichtet und ausgebildet ist, dass Patientengas nicht aus dem Gebläseteil 6, insbesondere dem Gebläsekopf 9, zum Antriebsvorrichtung 12 gelangt. In manchen nicht beanspruchten Beispielen ist vorgesehen, dass ein solcher Übergang von Patientengas vom Gebläsekopf 9 zum Antriebsvorrichtung 12 zumindest nicht über die Kopplung 11 erfolgen kann.

Die Antriebsvorrichtung 12 wird beispielhaft über einen elektrischen Anschluss 13 mit Energie versorgt.

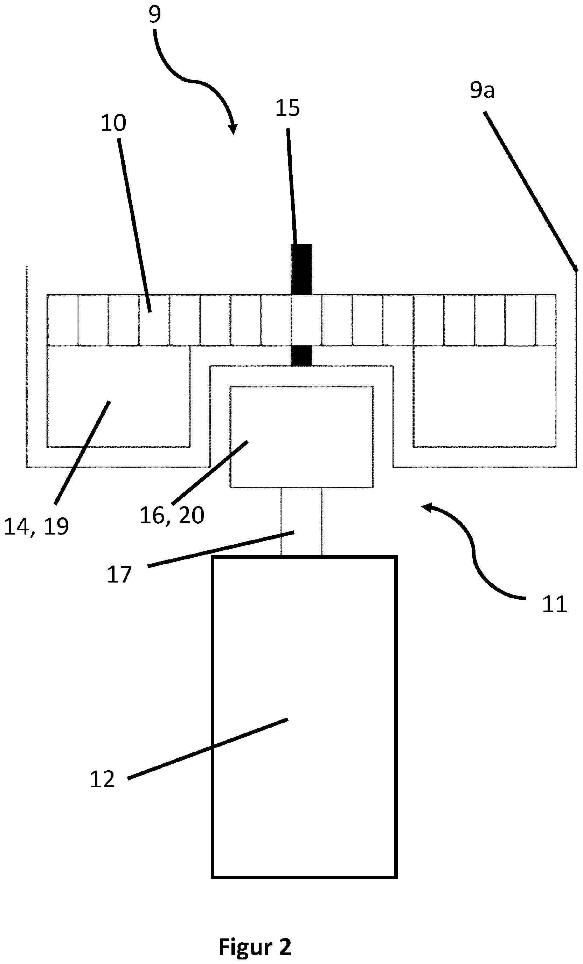

**Figur 2** zeigt eine schematische Detailansicht eines nicht beanspruchten Beispiel der Kopplung 11. Das gezeigte Beispiel stellt eine magnetische Kopplung, eine Art der kraftschlüssigen Verbindung, zwischen Antriebsvorrichtung 12 und Lüfterrad 10 dar. Als Koppelelemente 19, 20 dienen hierbei die Koppelmagneten 14, 16. Dazu ist an der mit der Antriebsvorrichtung 12 direkt verbundenen Motorachse 17 ein Koppelmagnet 16 angeordnet. Komplementär zum Koppelmagneten 16 der Motorachse 17 ist am Lüfterrad 10 zumindest ein Koppelmagnet 14 angeordnet. Die Koppelmagneten 14, 16 sind beispielsweise Permanentmagneten.

Das Lüfterrad 10 ist über ein Lager 15 drehbar in dem Gehäuse des Gebläsekopfes 9 gelagert. Die Kraftübertragung vom Antriebsvorrichtung 12 auf das Lüfterrad 10 erfolgt magnetisch über die Koppelmagneten 14, 16. In manchen nicht beanspruchten Beispielen ist im Gebläsekopf 9 eine feststehende Achse angeordnet, an welcher das Lüfterrad 10 drehbar gelagert ist. So kann beispielsweise eine einfache Lagerung über ein Kugellager erfolgen. In manchen Ausführungsformen kann vorgesehen sein, dass das Lager 15 in der Gehäusewand angeordnet ist und eine Achse die Verbindung zwischen Lager und Lüfterrad 10 herstellt.

Die beispielhafte Kopplung ist dabei so aufgebaut, dass der Koppelmagnet 14 des Lüfterrades 10 um den Koppelmagneten 16 der Motorachse 17 herum angeordnet ist. Beispielsweise ist der Kopplungsmanget 14 des Lüfterrades 10 als Hohlzylinder ausgebildet, welcher um den Koppelmagneten 16 der Motorachse 14 angeordnet ist. In manchen nicht beanspruchten Beispielen kann auch vorgesehen sein, dass am Lüfterrad 10 mehrere einzelne Koppelmagneten 14 angeordnet sind, welche in gleichmäßigen Abständen angeordnet sind. Durch die magnetische Kopplung ist kein materieller Kontakt zwischen Motorachse 17 und dem Lüfterrad 10 und/oder einer Lagerung und/oder einer Lüfterachse notwendig. Um zu verhindern, dass Patientengas aus dem Gebläsekopf 9 im Bereich der Kopplung 11 zum Motor bzw. der Antriebsvorrichtung 12 gelangen kann, ist beispielsweise vorgesehen, dass das Gehäuse des Gebläsekopfes 9 ohne Durchbruch oder anderen Öffnung zwischen Antriebsvorrichtung 12 (mit Motorachse 17 und Koppelmagnet 16) und dem Lüfterrad 10 inklusive Lagerung 15 etc. verläuft. In manchen nicht beanspruchten Beispielen ist vorgesehen, dass die Kopplung 11 hermetisch dicht ist.

Wie in Figur 2 zu erkennen ist, kann vorgesehen sein, dass das Gehäuse des Gebläsekopfes 9, somit auch das Gebläseteil 6, eine entsprechende Aussparung und/oder Einbuchtung aufweist, in welche zumindest der Kopplungsmanget 16 der Motorachse 17 zumindest teilweise eingeführt werden kann. Zwischen beiden Koppelmagneten 14, 16 verläuft die Gehäusewand des Gebläsekopfs 9 und trennt die Antriebsvorrichtung 12 und das Lüfterrad 10 zumindest im Bereich der Kopplung 11 hermetisch voneinander ab.

Durch die magnetische Kopplung 11 kann eine klare Trennung zwischen einem Bereich, welcher nicht mit Patientengas kontaminiert werden soll und einem mit Patientengas kontaminierten Bereich erreicht werden, bei der kein Patientengas aus dem kontaminierten Bereich in den nicht kontaminierten Bereich gelangt. Der Bereich, welcher nicht kontaminiert werden soll umfasst beispielsweise den Antriebsvorrichtung 12, während der kontaminierte Bereich das Lüfterrad 10 umfasst.

Die Trägheit des Lüfterrades 10 kann unter anderem durch die Größe der/des verwendeten Koppelmagneten 14 eingestellt werden. Auch die Positionierung entlang des Radius des Lüfterrades kann über die Trägheit des Lüfterrades 10 entscheidend sein.

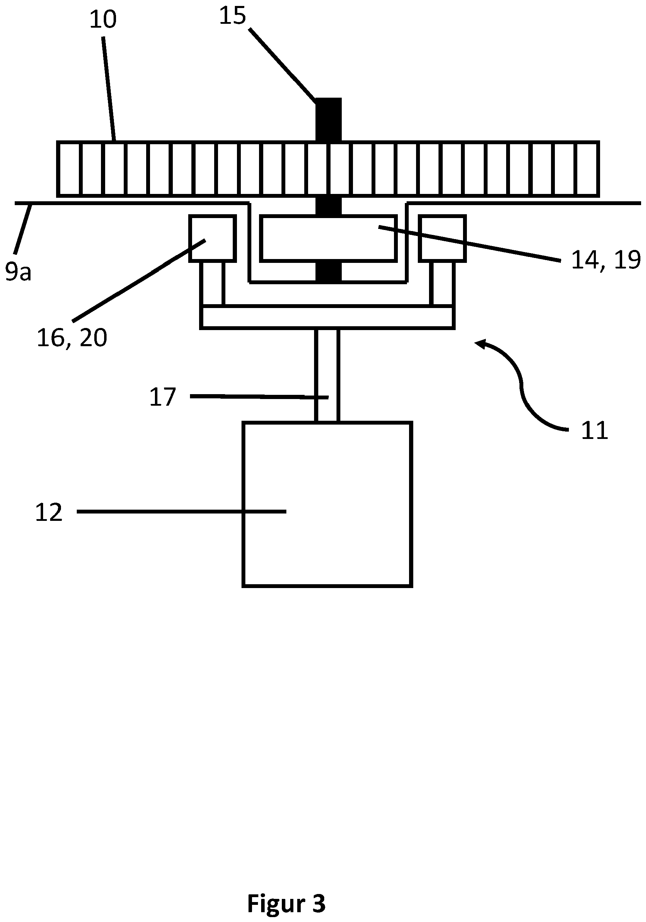

**Figur 3** zeigt ein nicht beanspruchtes Beispiel einer magnetischen Kopplung 11 bei welcher der Kopplungsmanget 16 der Motorachse 17 um den Koppelmagnet 14 des Lüfterrades 10 angeordnet ist. Der Koppelmagnet 14 des Lüfterrades 10 liegt also innerhalb des Koppelmagneten 16 der Motorachse 17. Das Lüfterrad 10 ist beispielhaft über das Lager 15 drehbar gelagert. Durch die Koppelmagneten 14, 16 wird eine Rotation der Motorachse 17 auf das Lager 15 und/oder direkt auf das Lüfterrad 10 übertragen. Der Koppelmagnet 16 der Motorachse ist beispielhaft als Hohlzylinder ausgebildet. Alternativ oder ergänzend kann der Koppelmagnet 16 auch aus einer Mehrzahl an Koppelmagneten bestehen, welche gleichmäßig um den Koppelmagneten 14 des Lüfterrades 10 angeordnet sind.

Der Gebläsekopf 9 und das den Gebläsekopf 9 beinhaltende Gebläseteil 6 weisen beispielhaft eine Ausbuchtung auf, welche vom Koppelmagneten 16 der Motorachse 17 umgeben werden kann.

In dem Beispiel verläuft das Gehäuse des Gebläsekopfes 9 zwischen dem Koppelmagneten 14 des Lüfterrades 10 und dem Koppelmagneten 16 der Motorachse 17.

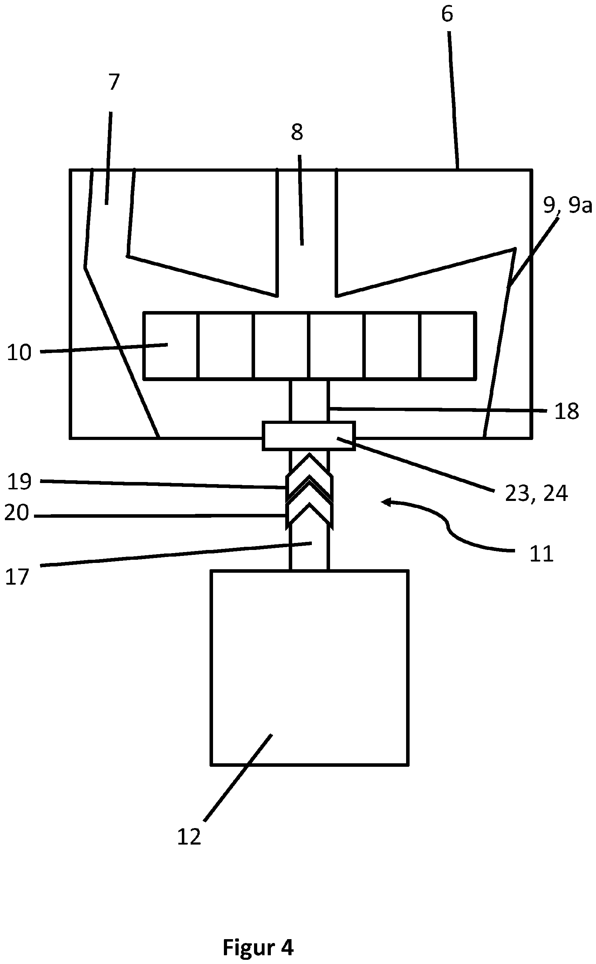

In **Figur 4** ist die erfinderische Ausführungsform einer kraftschlüssigen Verbindung als lösbare Kopplung 11 schematisch dargestellt. Das Lüfterrad 10 ist mit einer Lüfterachse 18 verbunden. An einem Ende der Lüfterachse 18 ist ein Koppelelement 19 angeordnet. Die Lüfterachse 18 ist beispielhaft im Gehäuse des Gebläsekopfes 9 bzw. des Gebläseteils 6 über das Lager 24 gelagert. Das Lager 24 ist kombiniert mit einer Dichtung 23, welche dazu ausgebildet und eingerichtet ist, zu verhindern, dass Patientengas aus dem Gebläsekopf 9 zur Antriebsvorrichtung 12 gelangt.

Die Motorachse 17 verfügt über zu der Koppelelement 19 der Lüfterachse 18 komplenentäres Koppelelement 20. Beispielsweise wird über die Koppelelemente 19, 20 ein Formschluss erreicht. Hierbei können asymmetrische oder symmetrische Profile der Koppelelemente 19, 20 verwendet werden. Für eine einfache Montage können zusätzlich noch Führungselemente vorgesehen sein, an denen entlang beispielsweise das Gebläseteil 6 geführt wird. Die Führungselemente führen das Gebläseteil 6 dann so an die Antriebsvorrichtung 12 heran, dass die beiden Koppelelemente 19, 20 ineinandergreifen bzw. sich verbinden. In manchen Ausführunsformen ist ergänzend zum Kraftschluss ein Formschluss vorgesehen. Der Kraftschluss kann beispielweise ein Reibschluss sein.

Die Koppelelemente 19, 20 werden so aneinander gedrückt/gepresst, dass eine Kraftübertragung von Antriebsvorrichtung 12 bzw. der Motorachse 17 an das Lüfterrad 10 bzw. die Lüfterachse 18 erfolgt. Erfindungsgemäß weisen die Koppelelemente 19, 20 eine konische Form auf. Beispielsweise ist das Koppelelement 19 als Hohlkegel ausgeführt und das Koppelelement 20 als Kegel, welcher in den Hohlkegel des Koppelelements 19 eingeführt werden kann.

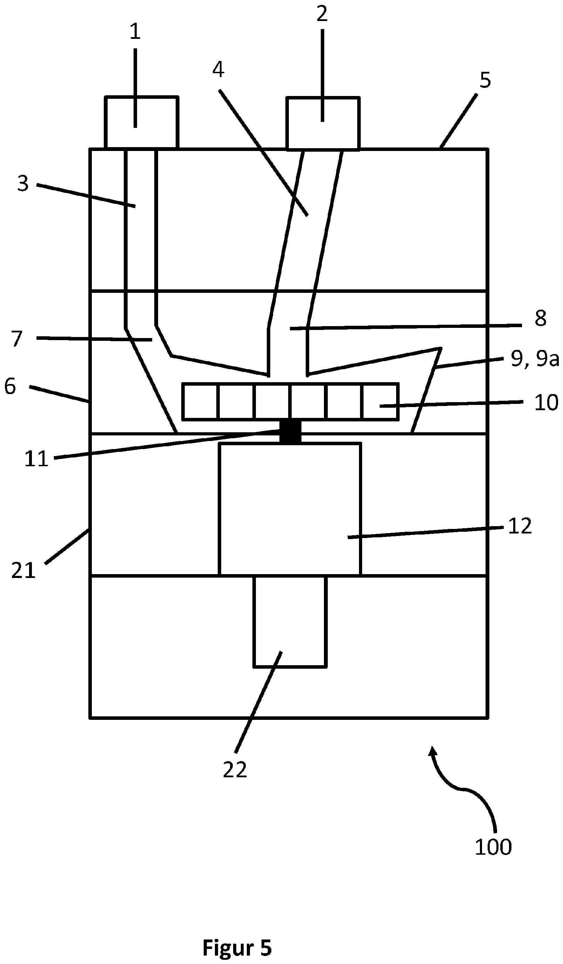

In **Figur 5** ist ein Ausschnitt aus einem beispielhaften Beatmungsgerät schematisch dargestellt. Neben den zuvor beschriebenen entnehmbaren Gebläseteil 6 und Gasführungsteil 5 ist in dem gezeigten Beispiel auch ein separat entnehmbares Motorteil 21 vorgesehen. Das Motorteil 21 umfasst zumindest einen Motor bzw. die Antriebsvorrichtung 12. Die Kopplung 11 zwischen Antriebsvorrichtung 12 und Lüfterrad 10 entsprich einer vorhergehend beschriebenen lösbaren Kopplung 11, ausgebildet als kraftschlüssige und/oder formschlüssige Verbindung. Beispielhaft ist die Kopplung 11 eine magnetische Kopplung.

Die Energieversorgung der Antriebsvorrichtung 12 erfolgt beispielsweise über eine Induktive Kopplung zwischen der Energiequelle 22 und der Antriebsvorrichtung 12. Dadurch kann die Antriebsvorrichtung 12 beispielhaft in einem hermetisch dichten Gehäuse angeordnet sein. Die Kraftübertragung zwischen Antriebsvorrichtung 12 und Lüfterrad 10 kann zum Beispiel ebenfalls magnetisch erfolgen. Alternativ oder ergänzend kann vorgesehen sein, dass die Motorachse 17 der Antriebsvorrichtung 12 aus dem Gehäuse des Motorteils 21 herausragt und in der Gehäusewand des Motorteils 21 gelagert ist. Die Lagerung in dem Gehäuse des Motorteils 21 ist dabei als hermetisch dicht vorgesehen, sodass kein Patientengas in das Motorteil 21, zumindest aber nicht zur Antriebsvorrichtung 12 gelangen kann.

Eine solche modulare Bauweise, bei der zum Beispiel verschiedene Arten und Typen an Motorteil 21, Gebläseteil 6 und Gasführungsteil 5 zur Verfügung stehen, ermöglicht eine einfache Anpassung des Beatmungsgerätes an einen aktuellen Bedarf, ohne dass mehrere Beatmungsgeräte unterschiedlicher Bauweise vorgehalten werden müssen.

### Bezugszeichenliste

1 Exspirationszweig
2 Inspirationszweig
3 Gasführung
4 Gasführung
5 Gasführungsteil
6 Gebläseteil
7 Gebläsestutzen
8 Gebläsestutzen
9 Gebläsekopf
9a Gehäuse
10 Lüfterrad
11 Kopplung
12 Antriebsvorrichtung
13 elektrischer Anschluss
14 Erster Koppelmagnet
15 Lager
16 Zweiter Koppelmagnet
17 Motorachse
18 Lüfterachse
19 Zweites Koppelelement
20 Erstes Koppelelement
21 Motorteil
22 Energiequelle
23 Dichtung
24 Lager
100 Beatmungsgerät

## Patentansprüche

1. Beatmungsgerät (100) mit einem Gebläsekopf (9) und einer Antriebsvorrichtung (12), wobei der Gebläsekopf (9) zumindest ein Lüfterrad (10) und ein Gehäuse (9a) mit Gebläsestutzen (7, 8) umfasst und zusammen mit der Antriebsvorrichtung (12) dazu ausgebildet ist, ein Patientengas zu fördern, wobei der Gebläsekopf (9) und die Antriebsvorrichtung (12) über eine Kopplung (11) miteinander lösbar gekoppelt sind, wobei das Lüfterrad (10) mit einer Lüfterachse (18) verbunden ist, an deren Ende ein erstes Koppelelement (19) angeordnet ist, wobei eine Motorachse (17) der Antriebsvorrichtung (12) über ein zum ersten Koppelelement (19) komplementäres zweites Koppelelement (20) verfügt,
**dadurch gekennzeichnet, dass** die Koppelelemente (19, 20) für einen Kraftschluss, bei dem die Koppelelemente (19, 20) so aneinandergedrückt werden, dass eine Kraftübertragung von der Motorachse (17) auf die Lüfterachse (18) erfolgt, eine konische Form aufweisen.

2. Beatmungsgerät (100) nach Anspruch 1, wobei die Lüfterachse (18) im Gehäuse (9a) über ein mit einer Dichtung (23) kombiniertes Lager (24) gelagert ist, wobei die Dichtung (23) dazu ausgebildet und eingerichtet ist, zu verhindern, dass das Patientengas aus dem Gebläsekopf (9) zur Antriebsvorrichtung (12) gelangt.

3. Beatmungsgerät (100) nach einem der vorhergehenden Ansprüche, wobei das erste Koppelelement (19) als ein Hohlkegel ausgeführt ist und das zweite Koppelelement (20) als ein in den Hohlkegel einführbarer Kegel ausgeführt ist.

4. Beatmungsgerät (100) nach einem der vorhergehenden Ansprüche, wobei zusätzlich Führungselemente vorgesehen sind, entlang derer ein Gebläseteil (6) an die Antriebsvorrichtung (12) herangeführt werden kann, sodass die beiden Koppelelemente (19, 20) ineinandergreifen oder sich verbinden.

5. Beatmungsgerät (100) nach einem der vorhergehenden Ansprüche, wobei der Gebläsekopf (9) als Verschleiß- oder Aufbereitungsteil ausgebildet ist und für eine Aufbereitung in einem Thermodesinfektor und/oder einem Autoklav ausgelegt ist.

6. Beatmungsgerät (100) nach einem der vorhergehenden Ansprüche, wobei die Koppelelemente (19, 20) ein symmetrisches oder asymmetrisches Kopplungsprofil zum zusätzlichen Herstellen einer formschlüssigen Verbindung aufweisen.

## Claims

1. A breathing device (100) with a fan head (9) and a drive device (12), wherein the fan head (9) comprises at least one fan wheel (10) and a housing (9a) with fan connecting pieces (7, 8) and is designed, together with the drive device (12), to convey a patient gas, wherein the fan head (9) and the drive device (12) are releasably coupled to each other via a coupling (11), wherein the fan wheel (10) is connected to a fan shaft (18), on the end of which a first coupling element (19) is arranged, wherein a motor shaft (17) of the drive device (12) has a second coupling element (20) that is complementary to the first coupling element (19), **characterized in that** the coupling elements (19, 20) have a conical shape for a force-fit, in which the coupling elements (19, 20) are pressed against each other such that force is transmitted from the motor shaft (17) to the fan shaft (18).

2. The breathing device (100) according to claim 1, wherein the fan shaft (18) is mounted in the housing (9a) by a bearing (24) combined with a seal (23), wherein the seal (23) is designed and configured to prevent the patient gas from escaping from the fan head (9) to the drive device (12).

3. The breathing device (100) according to one of the preceding claims, wherein the first coupling element (19) is designed as a hollow cone and the second coupling element (20) is designed as a cone that can be inserted into the hollow cone.

4. The breathing device (100) according to one of the preceding claims, wherein guide elements are additionally provided, along which a fan part (6) can be guided toward the drive device (12) so that the two coupling elements (19, 20) can engage with each other or connect.

5. The breathing device (100) according to one of the preceding claims, wherein the fan head (9) is designed as a wear or treatment part and is designed for treatment in a thermal disinfector and/or an autoclave.

6. The breathing device (100) according to one of the preceding claims, wherein the coupling elements (19, 20) have a symmetrical or asymmetrical coupling profile for additionally establishing a form-fitting connection.

## Revendications

1. Dispositif respiratoire (100) doté d'une tête de ventilateur (9) et d'un système d'entraînement (12), dans lequel la tête de ventilateur (9) comporte au moins une roue de soufflante (10) et un boîtier (9a) comprenant des tubulures de ventilateur (7, 8) et est conçue pour transporter un gaz de patient, conjointement avec le système d'entraînement (12), dans lequel la tête de ventilateur (9) et le système d'entraînement (12) sont accouplés l'un à l'autre de façon détachable par le biais d'un accouplement (11), dans lequel la roue de soufflante (10) est reliée à un axe de soufflante (18), à l'extrémité duquel est disposé un premier élément d'accouplement (19), dans lequel un axe de moteur (17) du système d'entraînement (12) dispose d'un deuxième élément d'accouplement (20) complémentaire au premier élément d'accouplement (19), **caractérisé en ce que** les éléments d'accouplement (19, 20) présentent une forme conique pour un assemblage à force, où les éléments d'accouplement (19, 20) sont pressés l'un contre l'autre de manière à réaliser une transmission de force de l'axe de moteur (17) à l'axe de soufflante (18).

2. Dispositif respiratoire (100) selon la revendication 1, dans lequel l'axe de soufflante (18) est monté dans le boîtier (9a) par le biais d'un palier (24) combiné à un joint d'étanchéité (23), dans lequel le joint d'étanchéité (23) est conçu et configuré pour empêcher que le gaz de patient ne s'échappe de la tête de ventilateur (9) vers le système d'entraînement (12).

3. Dispositif respiratoire (100) selon l'une des revendications précédentes, dans lequel le premier élément d'accouplement (19) est réalisé comme un cône creux et le deuxième élément d'accouplement (20) est réalisé comme un cône apte à être inséré dans le cône creux.

4. Dispositif respiratoire (100) selon l'une des revendications précédentes, dans lequel il est en outre prévu des éléments de guidage, le long desquels une pièce de ventilateur (6) peut être rapproché du système d'entraînement (12), de sorte que les deux éléments d'accouplement (19, 20) s'engagent l'un dans l'autre ou se relient.

5. Dispositif respiratoire (100) selon l'une des revendications précédentes, dans lequel la tête de ventilateur (9) est conçue comme une pièce d'usure ou de traitement et adaptée à un traitement dans un désinfecteur thermique et/ou un autoclave.

6. Dispositif respiratoire (100) selon l'une des revendications précédentes, dans lequel les éléments d'accouplement (19, 20) présentent un profil d'accouplement symétrique ou asymétrique pour la création supplémentaire d'une liaison à complémentarité de forme.
